# EUROPÄISCHE PATENTSCHRIFT

(11) **EP 3 538 898 B1**
(45) Veröffentlichungstag und Bekanntmachung des Hinweises auf die Patenterteilung: **04.12.2019**
(21) Anmeldenummer: 18815546.9
(22) Anmeldetag: 27.11.2018
(51) Int. Cl.: G01N 33/86

(54) **PROTHROMBINZEIT-REAGENZ ENTHALTEND EINEN EISENCHELATOR**
PROTHROMBIN TIME REAGENT COMPRISING AN IRON CHELATOR
RÉACTIF POUR LE TEMPS DE PROTHROMBINE CONTENANT UN CHÉLATEUR DU FER

(30) Priorität: 28.11.2017 EP 17204000
(43) Veröffentlichungstag der Anmeldung: 18.09.2019
(73) Patentinhaber: Siemens Healthcare Diagnostics Products GmbH, 35041 Marburg (DE)
(72) Erfinder: PILGRIM, Sabine, 35037 Marburg (DE); WISSEL, Thomas, 35094 Lahntal (DE); ZANDER, Norbert, 35039 Marburg (DE)
(86) Internationale Anmeldenummer: PCT/EP2018/082632
(87) Internationale Veröffentlichungsnummer: WO 2019/105907

(56) Entgegenhaltungen:
- EP-A2- 0 942 284
- WO-A1-98/49562
- WO-A1-2017/139567
- ROSSI N A A ET AL: "In vitro chelating, cytotoxicity, and blood compatibility of degradable poly(ethylene glycol)-based macromolecular iron chelators", BIOMATERIALS, ELSEVIER SCIENCE PUBLISHERS BV., BARKING, GB, Bd. 30, Nr. 4, 1. Februar 2009 (2009-02-01), Seiten 638-648, XP025693622, ISSN: 0142-9612, DOI: 10.1016/J.BIOMATERIALS.2008.09.057 [gefunden am 2008-11-01]
- ZU D. LIU ET AL: "Design of clinically useful iron(III)-selective chelators", MEDICINAL RESEARCH REVIEWS, Bd. 22, Nr. 1, 1. Januar 2001 (2001-01-01) , Seiten 26-64, XP055210372, ISSN: 0198-6325, DOI: 10.1002/med.1027

## Beschreibung

Die vorliegende Erfindung liegt auf dem Gebiet der Gerinnungsdiagnostik und betrifft ein Prothrombinzeit-Reagenz auf Basis von rekombinantem oder nativem Gewebefaktorprotein und Phospholipiden, welches durch die Zugabe eines Eisenchelators ausgewählt aus der Gruppe Siderophor und 3,5-Diphenyl-1,2,4-Triazol stabilisiert werden kann.

Das Gewebefaktorprotein (kurz: Gewebefaktor oder Thromboplastin, engl. tissue factor) ist ein Transmembranprotein mit essentieller Bedeutung für die Blutgerinnung. Es wird von Zellen exprimiert, die normalerweise nicht mit fließendem Blut in Kontakt stehen, wie z.B. von Zellen im Subendothel (Glatte Muskulatur) und von Zellen, die Blutgefäße umgeben (z.B. Fibroblasten). Im Falle einer Blutgefäßschädigung geraten die Gewebefaktorprotein-exprimierenden Zellen jedoch in Kontakt mit Faktor VII, einem prokoagulatorischen Blutgerinnungsfaktor, der im Blut zirkuliert. In Gegenwart von Calcium bilden Gewebefaktorprotein und Faktor VII einen Komplex, und die Aktivität von Faktor VII wird vertausendfacht (F VII > F VIIa). Der Komplex aus Gewebefaktorprotein und Faktor VIIa katalysiert in Gegenwart von Phospholipiden und Calcium die Umwandlung des inaktiven Blutgerinnungsfaktors Faktor X in aktivierten Faktor Xa und beschleunigt so den Gerinnungsprozess. Zusammen mit Faktor VII bildet der Gewebefaktor den sogenannten extrinsischen Weg der Blutgerinnung, durch den eine Verletzung der Blutgefäße durch schnellstmögliche Blutgerinnung unterbunden werden soll.

In der Gerinnungsdiagnostik wurden verschiedene in vitro-Testverfahren entwickelt, mit deren Hilfe bestimmt werden kann, ob das Blut oder Plasma eines Patienten einwandfrei gerinnen kann oder ob eine Gerinnungsstörung vorliegt. Im Fall einer Gerinnungsstörung ist es häufig erforderlich, präzisere Kenntnisse über die Ursache der vorliegenden Störung zu erlangen, um die optimalen therapeutischen Maßnahmen auswählen zu können. Zur Untersuchung verschiedener Teilfunktionen der Blutgerinnung, insbesondere zur Untersuchung des extrinsischen Systems der Blutgerinnung, wird Gewebefaktorprotein als Aktivator verwendet. Die bekannteste Anwendung von Gewebefaktorprotein als Gerinnungsaktivator ist der sogenannte Quick-Test zur Bestimmung der Prothrombinzeit (PT) (synonym: Thromboplastinzeit). Im Quick-Test und seinen Varianten wird üblicherweise eine Plasmaprobe mit einer Mischung von Gewebefaktorprotein, Phospholipiden und Calciumionen vermischt, und es wird die Zeit vom Zeitpunkt des Vermischens bis zur fassbaren Fibrinbildung in Sekunden gemessen. In Gerinnungstesten, bei denen chromogene Substrate verwendet werden, wird alternativ die Zeit vom Zeitpunkt des Vermischens bis zum Erreichen einer bestimmten Absorptionsänderung gemessen. Gewebefaktorprotein wird auch in anderen Testverfahren eingesetzt, die nicht der Bestimmung einer Gerinnungszeit dienen, sondern der Bestimmung einzelner Komponenten des Gerinnungssystems, wie z.B. des endogenen Thrombinpotenzials (ETP) (EP-A2-420332). Prinzipiell kann Gewebefaktorprotein bei allen Testen verwendet werden, die sich mit Komponenten der extrinsischen Gerinnung beschäftigen.

Das Prothrombinzeit-Reagenz (Gewebefaktor-Reagenz, PT-Reagenz) hat eine zentrale Bedeutung für den jeweiligen Test. Üblicherweise enthält ein Prothrombinzeit-Reagenz den Gewebefaktor zusammen mit gerinnungsaktiven Phospholipiden. Das Gewebefaktorprotein wird entweder als Gewebeextrakt aus unterschiedlichen Organen (z.B. Hirn, Plazenta, Lunge) verschiedener Spezies (z.B. Kaninchen, Mensch, Rind) gewonnen oder rekombinant hergestellt. Im Stand der Technik sind zahlreiche Methoden zur Gewinnung von Gewebefaktorprotein und zur Herstellung von Prothrombinzeit-Reagenzien bekannt, und eine Vielzahl von Prothrombinzeit-Reagenzien ist kommerziell erhältlich.

Zurzeit werden die meisten käuflichen Prothrombinzeit-Reagenzien in gefriergetrockneter Form gehandelt und müssen daher vor Gebrauch in einem Rekonstitutionsmedium, z.B. in destilliertem Wasser oder einer Pufferlösung aufgelöst werden. Der Grund hierfür ist die mangelnde Stabilität der Reagenzien im flüssigen Zustand. Der Nachteil von Reagenzien, die in gefriergetrockneter Form bereit gestellt werden, besteht nicht nur darin, dass Hersteller und Anwender zusätzliche zeit- und kostenintensive Verfahrensschritte durchführen müssen (Lyophilisation und Rekonstitution), sondern auch darin dass diese zusätzlichen Maßnahmen das Risiko bergen, dass es dabei zu Fehlern kommt, die die Qualität des Reagenzes beeinträchtigen können. Wünschenswert sind daher flüssige, gebrauchsfertige Reagenzformulierungen. Ein Problem bei der Bereitstellung flüssiger Prothrombinzeit-Reagenzien ist jedoch ihre mangelnde Stabilität. Unter der Stabilität eines Prothrombinzeit-Reagenzes kann z.B. die Beibehaltung der Prothrombinzeit für ein definiertes Plasma, z.B. ein Normalplasma, über die Lagerungzeit verstanden werden. Idealerweise sollte ein Prothrombinzeit-Reagenz über die Dauer seiner Lagerung oder seines Gebrauchs seine Spezifikationen beibehalten, beziehungsweise im günstigsten Fall die Eigenschaften und Charakteristika wie zum Zeitpunkt seiner Produktion.

Im Stand der Technik sind verschiedene Strategien zur Stabilisierung flüssiger Prothrombinzeit-Reagenzien beschrieben. In EP-A2-942284 wird ein flüssiges, auf rekombinantem Gewebefaktor basierendes Prothrombinzeit-Reagenz beschrieben, das durch die kombinierte Zugabe von Ascorbinsäure und einem Serumalbumin stabilisiert wird. In US 3,522,148 wird ein flüssiges, aus Gewebe extrahiertes (natürliches) Gewebefaktorprotein enthaltendes Prothrombinzeit-Reagenz beschrieben, das durch Zugabe bestimmter Natrium- oder Calciumsalze stabilisiert wird. In EP-A1-585987 wird ein anderes flüssiges, auf natürlichem Gewebefaktorprotein basierendes Prothrombinzeit-Reagenz beschrieben, das durch die Zugabe verschiedener Stabilisatoren, wie Albumin oder Polyethylenglykol, sowie verschiedener antimikrobiell wirksamer Substanzen, wie Natriumazid oder Antibiotika, stabilisiert wird. In EP-A2-524803 wird ein Verfahren zur Herstellung eines Prothrombinzeit-Reagenzes beschrieben, bei dem ein Metallionenchelator, insbesondere EDTA oder EGTA, bei der Extraktion des Gewebefaktors eingesetzt wird und im fertigen Reagenz enthalten sein kann. Die von dem Metallionenchelator gebundene Menge an Calciumionen wird durch Zugabe von zusätzlichem Calciumsalz ersetzt.

In US 2017/0234895 A1 ist ein zweiteiliges Kit zur Herstellung eines gebrauchsfertigen, flüssigen Prothrombinzeit-Reagenzes beschrieben, das in einem ersten Gefäß ein erste Lösung enthaltend Gewebefaktorprotein und Phospholipide enthält und in einem zweiten Gefäß eine Calciumchloridlösung enthält. In der ersten Lösung ist zum Zwecke der Stabilisierung ein Calciumchelator enthalten. Die beiden Lösungen werden erst kurz vor Gebrauch vom Anwender miteinander vermischt. Zwar kann hier seitens des Herstellers auf die Lyophilisation und seitens des Anwenders auf die Rekonstitutuion eines lyophilisierten Reagenzes verzichtet werden, allerdings muss der Anwender immer noch die beiden Flüssigkeiten miteinander vermischen, um das gebrauchsfertige Prothrombinzeit-Reagenz zu erhalten.

Der vorliegenden Erfindung lag also die Aufgabe zugrunde, ein im flüssigen Zustand langzeitstabiles, gebrauchsfertiges Prothrombinzeit-Reagenz, das mindestens Gewebefaktorprotein und Phospholipide enthält und das optional auch Calciumionen enthalten kann, bereit zu stellen.

Es wurde gefunden, dass die Zugabe eines Eisenchelators, insbesondere eines Eisenchelators aus der Gruppe der Siderophore und darunter insbesondere die Zugabe von Deferoxamin, Pyoverdin oder Ferrichrom oder die Zugabe eines Eisenchelators aus der Gruppe der 3,5-Diphenyl-1,2,4-Triazole und darunter insbesondere die Zugabe von Deferasirox, die Stabilität einer wässrigen Lösung, die Gewebefaktor und Phospholipide und optional Calciumionen enthält, erhöht. Erfindungsgemäß stabilisierte und im flüssigen Zustand gelagerte Prothrombinzeit-Reagenzien liefern noch nach 10-wöchiger Lagerung bei +37 °C Prothrombinzeit- (PT-) Messergebnisse, die weniger als 20 % von dem PT-Messergebnis zu Beginn der Laufzeit abweichen. Eine forcierte Lagerung bei +37 °C über 10 Wochen dient der schnellen Abschätzung, ob eine Lagerungsstabilität bei +2 °C bis +8 °C über einen Zeitraum von 12 oder mehr Monaten erwartet werden kann. Liefert ein stabilisiertes Prothrombinzeit-Reagenz bei einer Lagerung bei +37 °C über einen Zeitraum von 10 Wochen Prothrombinzeit- (PT-) Messergebnisse, die weniger als 20 % von dem PT-Messergebnis zu Beginn der Laufzeit abweichen, kann erwartet werden, dass das Reagenz bei einer Lagerungstemperatur von +2 °C bis +8 °C über einen Zeitraum von 12 oder mehr Monaten stabil ist.

Gegenstand der vorliegenden Erfindung ist also ein Prothrombinzeit-Reagenz enthaltend Gewebefaktorprotein und Phospholipide, das ferner mindestens einen Eisenchelator enthält, wobei der mindestens eine Eisenchelator ausgewählt ist aus der Gruppe Siderophor und 3,5-Diphenyl-1,2,4-Triazol.

Siderophore sind eine Klasse Eisen-bindender Chelatbildner, die von Mikroorganismen wie Bakterien und Pilzen und von Pflanzen gebildet werden und eine hohe Affinität für Eisen-III-Ionen (Fe³⁺-Ionen) aufweisen. Bevorzugte Siderophore sind Siderophore aus der Gruppe der 6-zähnigen Hydroxamate, wie z.B. Deferoxamin, Ferrichrom und Ferricrocin.

Bevorzugterweise ist der Eisenchelator aus der Gruppe der Siderophore ausgewählt aus der Gruppe Deferoxamin, Pyoverdin und Ferrichrom.

In einer Ausführungsform enthält ein erfindungsgemäßes Prothrombinzeit-Reagenz den Eisenchelator Deferoxamin aus der Gruppe der 6-zähnigen Hydroxamate (synonym: Desferrioxamin; Handelsname: Desferal), der beispielsweise von dem Bakterium Streptomyces pilosus gebildet wird.

In einer anderen Ausführungsform enthält ein erfindungsgemäßes Prothrombinzeit-Reagenz den Eisenchelator Pyoverdin, ein nichtribosomales Peptid, das beispielsweise von dem Bakterium Pseudomonas fluorescens gebildet wird und in die Gruppe der 6-zähnigen gemischten Hydroxamat- und Catecholate-Typus-Siderophore einzuordnen ist. Andere geeignete Eisenchelatoren aus der Gruppe der 6-zähnigen gemischten Hydroxamat- und Catecholate-Typus-Siderophore sind zum Beispiel Heterobactin und Yersinibactin.

In noch einer anderen Ausführungsform enthält ein erfindungsgemäßes Prothrombinzeit-Reagenz den Eisenchelator Ferrichrom, ein zyklisches Hexapeptid bestehend aus drei Glycin- und hydroylierten und acetylierten Ornithinresten (synonym: Deferriferrichrom), das beispielsweise von dem Pilz Ustilago sphaerogena gebildet wird und ebenfalls in die Gruppe der 6-zähnigen Hydroxamate einzuordnen ist.

3,5-Diphenyl-1,2,4-Triazole sind eine Klasse Eisen-bindender Chelatbildner, die u.a. zu therapeutischen Zwecken entwickelt wurden (EP-B1-0914118). Bevorzugte 3,5-Diphenyl-1,2,4-Triazole sind Deferasirox, Ethyl[3,5-bis(2-hydroxyphenyl)-[1,2,4]triazol-1-yl]acetat und 3,5-Bis(2-hydroxyphenyl)-1-(2,2,2-trifluoroethyl)-1H-[1,2,4]triazol.

In einer entsprechenden Ausführungsform enthält ein erfindungsgemäßes Prothrombinzeit-Reagenz einen Eisenchelator aus der Gruppe der 3,5-Diphenyl-1,2,4-Triazole und darunter insbesondere Deferasirox (IUPAC-Name: 4-[(3Z,5E)-3,5-bis(6-oxocyclohexa-2,4-dien-1-ylidene)-1,2,4-triazolidin-1-yl]benzoic acid), Ethyl[3,5-bis(2-hydroxyphenyl)-[1,2,4]triazol-1-yl]acetat oder 3,5-Bis(2-hydroxyphenyl)-1-(2,2,2-trifluoroethyl)-1H-[1,2,4]triazol.

Ein erfindungsgemäßes Prothrombinzeit-Reagenz enthält einen der vorgenannten geeigneten Eisenchelatoren vorzugsweise in einer Konzentration von 0,007 bis 2,5 mmol/L, besonders bevorzugt von 0,01 bis 0,5 mmol/L.

Ein erfindungsgemäßes Prothrombinzeit-Reagenz kann einen einzigen oder eine Kombination aus zwei, drei, vier oder mehr der vorgenannten geeigneten Eisenchelatoren enthalten.

In einer besonders bevorzugten Ausführungsform enthält ein erfindungsgemäßes Prothrombinzeit-Reagenz ferner Calciumionen. Zu diesem Zweck kann das Prothrombinzeit-Reagenz Calciumchlorid enthalten, vorzugsweise in einer Konzentration von 5 bis 500 mmol/L.

Das in dem Prothrombinzeit-Reagenz enthaltene Gewebefaktorprotein ist vorzugsweise ausgewählt aus der Gruppe humanes oder tierisches (z.B. Kaninchen, Rind etc.) rekombinantes Gewebefaktorprotein und natürliches humanes oder tierisches Gewebefaktorprotein aus einem Gewebeextrakt (z.B. aus Hirn, Plazenta, Lunge etc.).

Die Phospholipide können aus natürlichen und/oder synthetischen Quellen stammen.

Auch wenn der besondere Vorteil der vorliegenden Erfindung darin besteht, dass ein erfindungsgemäßes Prothrombinzeit-Reagenz als gebrauchsfertiges Flüssigreagenz gelagert werden kann, so ist es jedoch selbstverständlich auch möglich, dass das Reagenz als in Wasser oder Puffer rekonstituierbares Lyophilisat bereitgestellt wird.

Ein weiterer Gegenstand der vorliegenden Erfindung ist ein Verfahren zur Herstellung eines flüssigen Prothrombinzeit-Reagenzes, wobei eine Flüssigkeit enthaltend Gewebefaktorprotein, Phospholipide und mindestens einen Eisenchelator ausgewählt aus der Gruppe Siderophor, darunter bevorzugterweise Deferoxamin, Pyoverdin oder Ferrichrom, und 3,5-Diphenyl-1,2,4-Triazol, darunter vorzugsweise Deferasirox, Ethyl[3,5-bis(2-hydroxyphenyl)-[1,2,4]triazol-1-yl]acetat oder 3,5-Bis(2-hydroxyphenyl)-1-(2,2,2-trifluoroethyl)-1H-[1,2,4]triazol, bereit gestellt wird und in ein Reagenzgefäß abgefüllt wird, ohne dass diese Flüssigkeit lyophilisiert wird.

Noch ein weiterer Gegenstand der vorliegenden Erfindung ist ein Verfahren zur Stabilisierung eines Prothrombinzeit-Reagenzes enthaltend Gewebefaktorprotein und Phospholipide und optional Ca²⁺-Ionen, indem dem Reagenz mindestens ein Eisenchelator ausgewählt aus der Gruppe Siderophor, vorzugsweise Deferoxamin, Pyoverdin oder Ferrichrom, und 3,5-Diphenyl-1,2,4-Triazol, vorzugsweise Deferasirox, Ethyl[3,5-bis(2-hydroxyphenyl)-[1,2,4]triazol-1-yl]acetat oder 3,5-Bis(2-hydroxyphenyl)-1-(2,2,2-trifluoroethyl)-1H-[1,2,4]triazol, zugesetzt wird.

Vorzugsweise wird bei dem erfindungsgemäßen Stabilisierungsverfahren der Eisenchelator in einer Menge zugesetzt, so dass das Reagenz den Eisenchelator schließlich in einer Konzentration von 0,007 bis 2,5 mmol/L, besonders bevorzugt von 0,01 bis 0,5 mmol/L enthält.

Ein anderer Gegenstand der vorliegenden Erfindung ist die Verwendung eines erfindungsgemäßen Prothrombinzeit-Reagenzes in einem in vitro-Verfahren zur Bestimmung eines Gerinnungsparameters in einer Patientenprobe, insbesondere zur Bestimmung eines Gerinnungsparameters aus der Gruppe Prothrombinzeit (PT, auch Quick-Test) und deren Varianten, von der Prothrombinzeit abgeleitetes Fibrinogen und Endogenes Thrombinpotenzial (ETP). Das erfindungsgemäße Reagenz eignet sich zur Verwendung als Aktivator der Gerinnungskaskade, beispielsweise in Testverfahren, die auf der Detektion eines Fibringerinnsels beruhen wie auch in chromogenen oder fluorogenen Testverfahren.

Die Erfindung betrifft ferner ein Verfahren zur Bestimmung eines Gerinnungsparameters in einer Plasmaprobe, wobei die Plasmaprobe mit einem erfindungsgemäßen Prothrombinzeit-Reagenz zu einem Reaktionsgemisch vermischt wird und der Gerinnungsparameter in dem Reaktionsgemisch bestimmt wird. Bei dem Gerinnungsparameter kann es sich beispielsweise um die Prothombinzeit oder das endogene Thrombinpotenzial einer Probe, beispielsweise einer humanen Vollblut- oder Plasmaprobe handeln.

Zur Bestimmung der Prothombinzeit wird vorzugsweise die Absorptionsänderung des Reaktionsgemisches photometrisch gemessen, und es wird die Zeit vom Zeitpunkt des Vermischens des erfindungsgemäßen Prothrombinzeit-Reagenzes mit der Plasmaprobe bis zur Erreichung eines Schwellwertes bestimmt.

Zur Ableitung der Fibrinogenkonzentration aus der Prothrombinzeitbestimmung ("abgeleitetes Fibrinogen") wird die Absorptionszunahme des Reaktionsgemisches photometrisch gemessen; diese korreliert mit der Fibrinogenkonzentration.

Die Erfindung betrifft ferner die Verwendung eines Eisenchelators zur Herstellung eines Prothrombinzeit-Reagenzes enthaltend Gewebefaktorprotein und Phospholipide, wobei der Eisenchelator ausgewählt ist aus der Gruppe Siderophor und 3,5-Diphenyl-1,2,4-Triazol. Bevorzugt verwendete Eisenchelatoren aus der Gruppe der Siderophore sind ausgewählt aus der Gruppe Deferoxamin, Pyoverdin und Ferrichrom. Bevorzugt verwendete Eisenchelatoren aus der Gruppe der 3,5-Diphenyl-1,2,4-Triazole sind ausgewählt aus der Gruppe Deferasirox, Ethyl[3,5-bis(2-hydroxyphenyl)-[1,2,4]triazol-1-yl]acetat und 3,5-Bis(2-hydroxyphenyl)-1-(2,2,2-trifluoroethyl)-1H-[1,2,4]triazol.

### FIGURENBESCHREIBUNG

**Figur 1**
   zeigt die Veränderung der Prothrombinzeiten in Sekunden (PT [s]) des normalen Kontrollplasmas Ci-Trol 1 mit den mit Deferoxamin stabilisierten PT-Reagenzien (9 = 9 mg/L = 0,0137 mM Deferoxamin; 12 = 12 mg/L = 0,0183 mM Deferoxamin; 15 = 15 mg/L = 0,0228 mM Deferoxamin) im Vergleich zu dem nicht stabilisierten PT-Reagenz ohne Deferoxamin (0 = 0 mg/L = 0 mM Deferoxamin) und zu dem lyophilisiert gelagerten und zum jeweiligen Zeitpunkt frisch gelösten Referenzreagenz (R) über die Zeit (t) in Wochen. Die Reagenzien wurden bei einer Temperatur von 37 °C gelagert.
**Figur 2**
   zeigt die Veränderung der Prothrombinzeiten in Sekunden (PT [s]) des abnormalen Kontrollplasmas Ci-Trol 2 mit den mit Deferoxamin stabilisierten PT-Reagenzien (9 = 9 mg/L = 0,0137 mM Deferoxamin; 12 = 12 mg/L = 0,0183 mM Deferoxamin; 15 = 15 mg/L = 0,0228 mM Deferoxamin) im Vergleich zu dem nicht stabilisierten PT-Reagenz ohne Deferoxamin (0 = 0 mg/L = 0 mM Deferoxamin) und zu dem lyophilisiert gelagerten und zum jeweiligen Zeitpunkt frisch gelösten Referenzreagenz (R) über die Zeit (t) in Wochen. Die Reagenzien wurden bei einer Temperatur von 37 °C gelagert.
**Figur 3**
   zeigt die Veränderung der Prothrombinzeiten in Sekunden (PT [s]) des normalen Kontrollplasmas Ci-Trol 1 mit den mit Deferoxamin stabilisierten PT-Reagenzien (15 = 15 mg/L = 0,0228 mM Deferoxamin; 75 = 75 mg/L = 0,114 mM Deferoxamin; 150 = 150 mg/L = 0,228 mM Deferoxamin) im Vergleich zu dem nicht stabilisierten PT-Reagenz ohne Deferoxamin (0 = 0 mg/L = 0 mM Deferoxamin) über die Zeit (t) in Wochen. Die Reagenzien wurden bei einer Temperatur von 37 °C gelagert.
**Figur 4**
   zeigt die Veränderung der Prothrombinzeiten in Sekunden (PT [s]) des abnormalen Kontrollplasmas Ci-Trol 2 mit den mit Deferoxamin stabilisierten PT-Reagenzien (15 = 15 mg/L = 0,0228 mM Deferoxamin; 75 = 75 mg/L = 0,114 Deferoxamin; 150 = 150 mg/L = 0,223 mM Deferoxamin) im Vergleich zu dem nicht stabilisierten PT-Reagenz ohne Deferoxamin (0 = 0 mg/L = 0 mM Deferoxamin) über die Zeit (t) in Wochen. Die Reagenzien wurden bei einer Temperatur von 37 °C gelagert.

Die folgenden Ausführungsbeispiele dienen der Veranschaulichung der vorliegenden Erfindung und sind nicht als Einschränkung zu verstehen.

### BEISPIEL 1: Bestimmung der Stabilität eines flüssigen, mit Deferoxamin stabilisierten Prothrombinzeit-Reagenzes

Zum Zeitpunkt t₀ wurde einem flüssigen Prothrombinzeit-Reagenz, das rekombinantes humanes Gewebefaktorprotein, synthetische Phospholipide und Calciumionen enthielt, in verschiedenen Ansätzen Deferoxamin (Deferoxamine mesylate salt, Sigma-Aldrich Chemie GmbH, Steinheim, Deutschland) in der angegebenen Endkonzentration zugesetzt:
1. 0 mM Deferoxamin,
2. 0,0137 mM (9 mg/L) Deferoxamin,
3. 0,0183 mM (12 mg/L) Deferoxamin,
4. 0,0228 mM (15 mg/L) Deferoxamin,
5. 0,114 mM (75 mg/L) Deferoxamin,
6. 0,228 mM (150 mg/L) Deferoxamin.

Die verschiedenen Reagenzien wurden in einem automatischen Prothrombinzeit-Test (PT-Test) in einem Sysmex® CA-1500-Analysegerät (Sysmex Corp., Kobe, Japan) eingesetzt. Als Proben wurden folgende definierte Kontrollplasmen eingesetzt:
- Dade® Ci-Trol® Coagulation Control Level 1 (Ci-Trol 1) Kontrollplasma; normale Kontrolle für die Bestimmung der Prothrombinzeit; die Ergebnisse sind mit denen mit frischem normalem Citratplasma vergleichbar;
- Dade® Ci-Trol® Coagulation Control Level 2 (Ci-Trol 2) Kontrollplasma; abnormale Kontrolle für die Bestimmung der Prothrombinzeit wie sie bei Gerinnungsstörungen zu beobachten sind; die Gerinnungszeiten sind gegenüber denen mit frischem normalem Citratplasma verlängert.

Probe und Reagenz wurden jeweils auf 37 °C vorgewärmt und schließlich vermischt. Durch die Zugabe des Reagenzes wurde der Gerinnungsvorgang ausgelöst, und es wurde die Zeit bis zur Bildung des Fibringerinnsels gemessen (Prothrombinzeit in Sekunden).

Zur Bestimmung der Langzeitstabilität der verschiedenen Reagenzien wurden die Reagenzien in flüssigem Zustand in mit einem Stopfen verschlossenen Glasflaschen bei +37 °C über einen Zeitraum von 16-18 Wochen gelagert. Etwa zweiwöchentlich wurden Proben der Reagenzien entnommen, und es wurde die Prothrombinzeit desselben Referenzplasmas bestimmt. Als Referenzreagenz wurde zu jedem Zeitpunkt ein lyophilisiertes Prothrombinzeit-Reagenz (Dade® Innovin® Reagenz, Siemens Healthcare Diagnostics Products GmbH, Deutschland) mit destilliertem Wasser frisch rekonstituiert und gemessen.

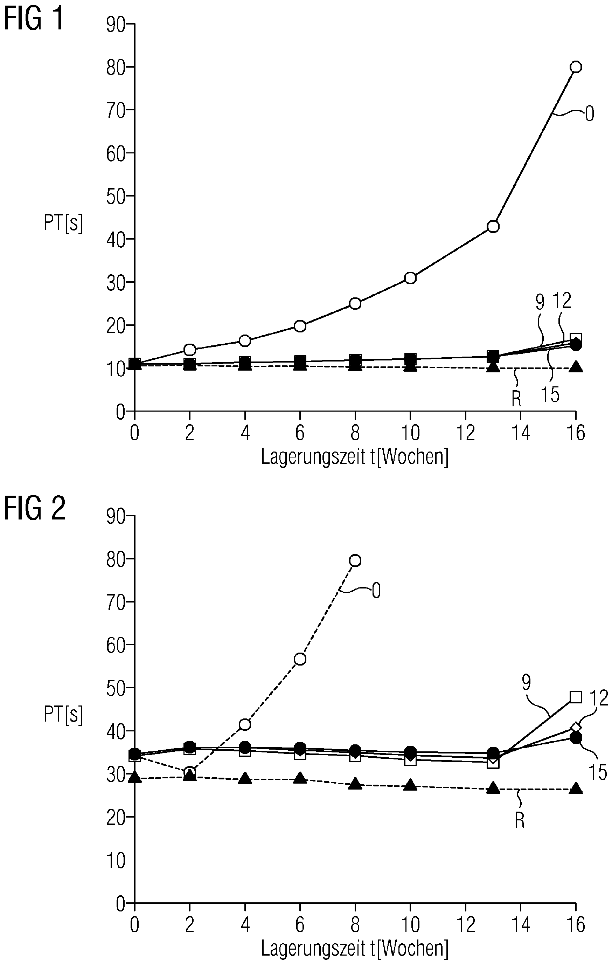

In Figur 1 (Ci-Trol 1 als Probe) und Figur 2 (Ci-Trol 2 als Probe) sind die Prothrombinzeiten der verschiedenen Prothrombinzeit-Reagenzien (0, 12, 15 mg/L Deferoxamin) über einen Zeitraum von 16 Wochen bei +37 °C gezeigt. Stabile Prothrombinzeiten über einen Zeitraum von mindestens 10 Wochen mit Abweichungen von weniger als 20 % gegenüber dem Ausgangswert zum Zeitpunkt t₀ wurden nur mit dem lyophilisierten und jeweils frisch gelösten Referenzreagenz (R) und mit den erfindungsgemäßen, mit Deferoxamin stabilisierten Prothrombinzeit-Reagenzien erreicht. Das flüssig gelagerte Reagenz ohne Deferoxamin lieferte spätestens nach 4 Wochen nur noch stark abweichende Prothrombinzeiten.

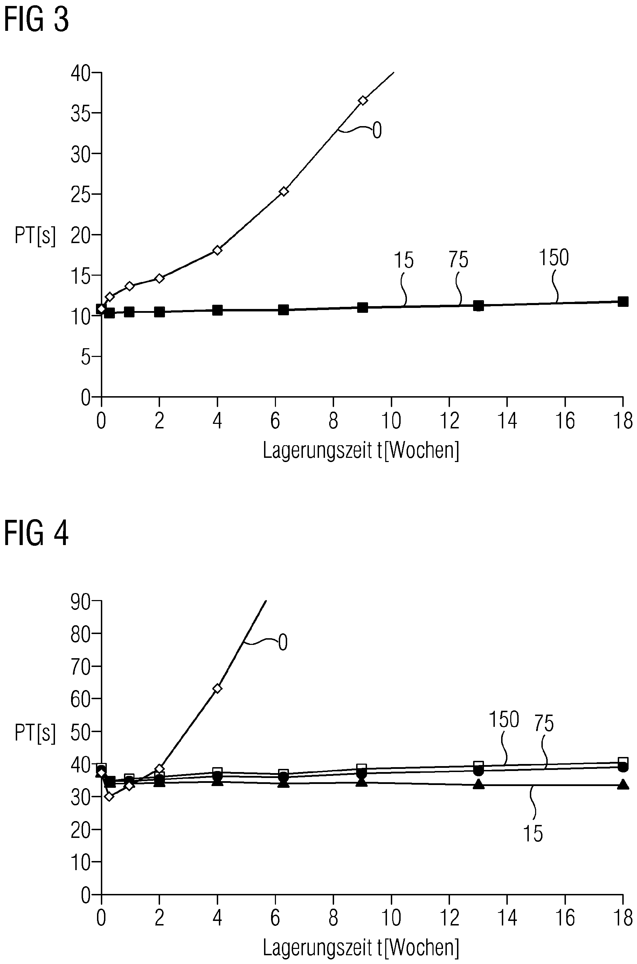

In Figur 3 (Ci-Trol 1 als Probe) und Figur 4 (Ci-Trol 2 als Probe) sind die Prothrombinzeiten der verschiedenen Prothrombinzeit-Reagenzien (15, 75, 150 mg/L Deferoxamin) über einen Zeitraum von 18 Wochen bei +37 °C gezeigt. Stabile Prothrombinzeiten über einen Zeitraum von mindestens 10 Wochen mit Abweichungen von weniger als 20 % gegenüber dem Ausgangswert zum Zeitpunkt t₀ wurden nur mit den erfindungsgemäßen, mit Deferoxamin stabilisierten Prothrombinzeit-Reagenzien erreicht. Das flüssig gelagerte Reagenz ohne Deferoxamin lieferte spätestens nach 4 Wochen nur noch stark abweichende Prothrombinzeiten.

### BEISPIEL 2: Bestimmung der Stabilität flüssiger, mit verschiedenen Eisenchelatoren stabilisierter Prothrombinzeit-Reagenzien

Zum Zeitpunkt t₀ wurde einem flüssigen Prothrombinzeit-Reagenz, das rekombinantes humanes Gewebefaktorprotein, synthetische Phospholipide und Calciumionen enthielt, in verschiedenen Ansätzen jeweils 0,0228 mmol/L folgender Metallionenchelatoren zugesetzt:
i. Pyoverdin (Pyoverdines, Sigma-Aldrich Chemie GmbH, Steinheim, Deutschland);
ii. Ferrichrom (Ferrichrome Iron-free from Ustilago sphaerogena, Sigma-Aldrich Chemie GmbH, Steinheim, Deutschland);
iii. Deferoxamin (Deferoxamine mesylate salt, Sigma-Aldrich Chemie GmbH, Steinheim, Deutschland) Deferoxamin (Deferoxamine mesylate salt, Sigma-Aldrich Chemie GmbH, Steinheim, Deutschland);
iv. Deferasirox (Combi-Blocks, Inc., San Diego, USA);
v. DTPA (Diethylentriaminpentaessigsäure);
vi. EDTA (Ethylendiamintetraessigsäure);
vii. BAPTA (1,2-Bis(2-aminophenoxy)ethan-N,N,N',N'-tetraessigsäure).

Die verschiedenen Reagenzien wurden wie in Beispiel 1 beschrieben in einem automatischen Prothrombinzeit-Test (PT-Test) in einem Sysmex® CA-1500-Analysegerät (Sysmex Corp., Kobe, Japan) eingesetzt. Als Proben wurden die in Beispiel 1 näher beschriebenen Kontrollplasmen Ci-Trol 1 und Ci-Trol 2 eingesetzt.

Zur Bestimmung der Langzeitstabilität der verschiedenen Reagenzien wurden die Reagenzien in flüssigem Zustand in mit einem Stopfen verschlossenen Glasflaschen bei +37 °C über einen Zeitraum von 16-18 Wochen gelagert. Etwa monatlich wurden Proben der Reagenzien entnommen, und es wurde die Prothrombinzeit desselben Referenzplasmas bestimmt.

Die verschiedenen Messergebnisse (Prothrombinzeit in Sekunden, PT [s]) zum Zeitpunkt t₀, nach zwei- und nach viermonatiger Lagerung (2 M, 4 M) und die entsprechende Abweichung in % von der Prothrombinzeitmessung zum Zeitpunkt t₀ sind in Tabelle 1 (Ci-Trol 1-Kontrollplasma als Probe) und in Tabelle 2 (Ci-Trol 2-Kontrollplasma als Probe) gezeigt.

**Tabelle 1**

| **Chelator** | **t₀ PT [s]** | **2 M PT [s]** | **4 M PT [s]** | **2 M %** | **4 M %** |
|---|---|---|---|---|---|
| **Pyoverdin** | 12,0 | 11,8 | 13,4 | -1,7 | 11,7 |
| **Ferrichrom** | 12,1 | 11,9 | 12,9 | -1,7 | 6, 6 |
| **Deferoxamin** | 11,6 | 11,5 | 13,1 | -0,9 | 12,9 |
| **Deferasirox** | 11,6 | 11,4 | 13,8 | -1,7 | 19,0 |
| **DTPA** | 11,6 | 12,5 | 22,4 | 7,8 | 93,1 |
| **EDTA** | 11,8 | 35,9 | 71,5 | 204,2 | 505,9 |
| **BAPTA** | 11,6 | 40,2 | 72,8 | 246,6 | 527,6 |
| **Ohne Chelator** | 11,6 | 36,7 | 71,7 | 216,4 | 512,9 |

**Tabelle 2**

| **Chelator** | **t₀ PT [s]** | **2 M PT [s]** | **4 M PT [s]** | **2 M %** | **4 M %** |
|---|---|---|---|---|---|
| **Pyoverdin** | 43,5 | 37,6 | 38,7 | -13,6 | -11,0 |
| **Ferrichrom** | 44,4 | 38,8 | 40,0 | -12,6 | -9,9 |
| **Deferoxamin** | 39,5 | 36,3 | 37,1 | -8,1 | -6,1 |
| **Deferasirox** | 38,9 | 32,8 | 43,5 | -15,7 | 11,8 |
| **DTPA** | 39,0 | 39,1 | 82,0 | 0,3 | 110,3 |
| **EDTA** | 40,2 | 100,0 | 100,0 | 148,8 | 148,8 |
| **BAPTA** | 39,1 | 100,0 | 100,0 | 155,8 | 155,8 |
| **Ohne Chelator** | 38,8 | 100,0 | 100,0 | 157,7 | 157,7 |

Stabile Prothrombinzeiten über einen Zeitraum von vier Monaten (also mindestens 16 Wochen) mit Abweichungen von weniger als 20 % gegenüber dem Ausgangswert zum Zeitpunkt t₀ wurden nur mit den erfindungsgemäßen Eisenchelatoren, also mit Pyoverdin, Ferrichrom, Deferoxamin oder Deferasirox stabilisierten Prothrombinzeit-Reagenzien erreicht. Sowohl das flüssig gelagerte Reagenz ohne Chelator als auch die flüssig gelagerten Reagenzien, denen die aus dem Stand der Technik (US 2017/0234895 A1) bekannten Metallionenchelatoren DTPA, EDTA oder BAPTA zugegeben worden waren, lieferten spätestens nach 4 Monaten nur noch stark abweichende Prothrombinzeiten.

### BEISPIEL 3: Bestimmung der Stabilität flüssiger, mit verschiedenen Eisenchelatoren stabilisierter Prothrombinzeit-Reagenzien in Gegenwart erhöhter Fe³⁺-Ionen-Konzentrationen

Zum Zeitpunkt t₀ wurden einem flüssigen Prothrombinzeit-Reagenz, das rekombinantes humanes Gewebefaktorprotein, synthetische Phospholipide und Calciumionen enthielt, 0,00308 mM (0,5 mg/L) FeCl₃ zugesetzt und dann wurden in verschiedenen Ansätzen jeweils 0,0228 mM der in Beispiel 2 genannten Metallionenchelatoren zugesetzt.

Die verschiedenen Reagenzien wurden wie in Beispiel 1 beschrieben in einem automatischen Prothrombinzeit-Test (PT-Test) in einem Sysmex® CA-1500-Analysegerät (Sysmex Corp., Kobe, Japan) eingesetzt. Als Proben wurden die in Beispiel 1 näher beschriebenen Kontrollplasmen Ci-Trol 1 und Ci-Trol 2 eingesetzt.

Zur Bestimmung der Langzeitstabilität der verschiedenen Reagenzien in Gegenwart erhöhter Fe³⁺-Ionen-Konzentrationen wurden die Reagenzien in flüssigem Zustand in mit einem Stopfen verschlossenen Glasflaschen bei +37 °C über einen Zeitraum von 6 Wochen gelagert. Wöchentlich wurden Proben der Reagenzien entnommen, und es wurde die Prothrombinzeit desselben Referenzplasmas bestimmt.

Die verschiedenen Messergebnisse (Prothrombinzeit in Sekunden, PT [s]) zum Zeitpunkt t₀, nach ein-, zwei-, vier- und sechswöchiger Lagerung (1 W, 2 W, 4 W, 6 W) und die entsprechende Abweichung in % von der Prothrombinzeitmessung zum Zeitpunkt t₀ sind in Tabelle 3 (Ci-Trol 1-Kontrollplasma als Probe) und in Tabelle 4 (Ci-Trol 2-Kontrollplasma als Probe) gezeigt.

**Tabelle 3**

| **Chelator** | **t₀ PT [s]** | **1 W PT [s]** | **2 W PT [s]** | **4 W PT [s]** | **6 W PT [s]** | **1 W %** | **2 W %** | **4 W %** | **6 W %** |
|---|---|---|---|---|---|---|---|---|---|
| **Pyoverdin** | 12 | 11,2 | 11,4 | 11,5 | 11,4 | -6,7 | -5 | -4,2 | -5 |
| **Ferrichrom** | 12,1 | 11,2 | 11,4 | 11,6 | 11,6 | -7,4 | -5,8 | -4,1 | -4,1 |
| **Deferoxamin** | 11,4 | 10,7 | 11 | 11,1 | 11,2 | -6,1 | -3,5 | -2,6 | -1,8 |
| **Deferasirox** | 11,6 | 10,9 | 10,9 | 11,1 | 11,4 | -6 | -6 | -4,3 | -1,7 |
| **DTPA** | 11,8 | 13 | 13,4 | 14,9 | 17,7 | 10,2 | 13,6 | 26,3 | 50 |
| **EDTA** | 11,6 | 13 | 15,9 | 30,2 | 49,6 | 12,1 | 37,1 | 160,3 | 327,6 |
| **BAPTA** | 11,6 | 13,2 | 17,7 | 38,3 | 60,8 | 13,8 | 52,6 | 230,2 | 424,1 |
| **Ohne Chelator** | 11,4 | 13,1 | 17,9 | 48,9 | 59,5 | 14,9 | 57 | 328,9 | 421,9 |

**Tabelle 4**

| **Chelator** | **t₀ PT [s]** | **1 W PT [s]** | **2 W PT [s]** | **4 W PT [s]** | **6 W PT [s]** | **1 W %** | **2 W %** | **4 W %** | **6 W %** |
|---|---|---|---|---|---|---|---|---|---|
| **Pyoverdine** | 44 | 39,6 | 40 | 40,1 | 35,5 | -10 | -9,1 | -8,9 | -19,3 |
| **Ferrichrom** | 44,4 | 39,1 | 39,5 | 40,3 | 38,9 | -11,9 | -11 | -9,2 | -12,4 |
| **Deferoxamin** | 38 | 32,7 | 35,4 | 35,2 | 35,9 | -13,9 | -6,8 | -7,4 | -5,5 |
| **Deferasirox** | 39 | 35,1 | 33,5 | 31,2 | 32 | -10 | -14,1 | -20,0 | -17,9 |
| **DTPA** | 39,6 | 28,8 | 30,4 | 39,2 | 55,7 | -27,3 | -23,2 | -1 | 40,7 |
| **EDTA** | 38,6 | 30,6 | 50 | 100 | 100 | -20,7 | 29,5 | 159,1 | 159,1 |
| **BAPTA** | 38,6 | 32,7 | 61,8 | 100 | 100 | -15,3 | 60,1 | 159,1 | 159,1 |
| **Ohne Chelator** | 38,4 | 32,1 | 64,8 | 100 | 100 | -16,4 | 68,8 | 160,4 | 160,4 |

Stabile Prothrombinzeiten über einen Zeitraum von sechs Wochen mit Abweichungen von weniger als 20 % gegenüber dem Ausgangswert zum Zeitpunkt t₀ wurden in Gegenwart erhöhter Fe³⁺-Ionen-Konzentrationen nur mit den erfindungsgemäßen Eisenchelatoren, also mit Pyoverdin, Ferrichrom, Deferoxamin oder Deferasirox stabilisierten Prothrombinzeit-Reagenzien erreicht. Sowohl das flüssig gelagerte Reagenz ohne Chelator als auch die flüssig gelagerten Reagenzien, denen die aus dem Stand der Technik (US 2017/0234895 A1) bekannten Metallionenchelatoren DTPA, EDTA oder BAPTA zugegeben worden waren, lieferten spätestens nach 2 Wochen nur noch stark abweichende Prothrombinzeiten.

## Patentansprüche

1. Prothrombinzeit-Reagenz enthaltend Gewebefaktorprotein und Phospholipide **dadurch gekennzeichnet, dass** das Reagenz ferner mindestens einen Eisenchelator enthält, wobei der mindestens eine Eisenchelator ausgewählt ist aus der Gruppe Siderophor und 3,5-Diphenyl-1,2,4-Triazol.

2. Prothrombinzeit-Reagenz gemäß Anspruch 1, wobei das Siderophor ausgewählt ist aus der Gruppe Deferoxamin, Pyoverdin und Ferrichrom.

3. Prothrombinzeit-Reagenz gemäß Anspruch 1, wobei das 3,5-Diphenyl-1,2,4-Triazol ausgewählt ist aus der Gruppe Deferasirox, Ethyl[3,5-bis(2-hydroxyphenyl)-[1,2,4]triazol-1-yl]acetat und 3,5-Bis(2-hydroxyphenyl)-1-(2,2,2-trifluoroethyl)-1H-[1,2,4]triazol.

4. Prothrombinzeit-Reagenz gemäß einem der vorhergehenden Ansprüche, das den mindestens einen Eisenchelator in einer Konzentration von 0,007 bis 2,5 mmol/L, besonders bevorzugt von 0,01 bis 0,5 mmol/L enthält.

5. Prothrombinzeit-Reagenz gemäß einem der vorhergehenden Ansprüche, das ferner Ca²⁺-Ionen enthält.

6. Prothrombinzeit-Reagenz gemäß Anspruch 5, das 5 bis 500 mmol/L Calciumchlorid enthält.

7. Prothrombinzeit-Reagenz gemäß einem der vorhergehenden Ansprüche, wobei das Gewebefaktorprotein ausgewählt ist aus der Gruppe humanes oder tierisches rekombinantes Gewebefaktorprotein und natürliches humanes oder tierisches Gewebefaktorprotein aus einem Gewebeextrakt.

8. Verfahren zur Herstellung eines flüssigen Prothrombinzeit-Reagenzes enthaltend Gewebefaktorprotein und Phospholipide und optional Ca²⁺-Ionen **dadurch gekennzeichnet, dass** eine Flüssigkeit enthaltend Gewebefaktorprotein, Phospholipide und mindestens einen Eisenchelator ausgewählt aus der Gruppe Siderophor und 3,5-Diphenyl-1,2,4-Triazol bereit gestellt wird und die Flüssigkeit in ein Reagenzgefäß abgefüllt wird, ohne dass diese Flüssigkeit lyophilisiert wird.

9. Verfahren zur Stabilisierung eines Prothrombinzeit-Reagenzes enthaltend Gewebefaktorprotein und Phospholipide und optional Ca²⁺-Ionen **dadurch gekennzeichnet, dass** dem Reagenz mindestens ein Eisenchelator ausgewählt aus der Gruppe Siderophor und 3,5-Diphenyl-1,2,4-Triazol zugesetzt wird.

10. Verfahren gemäß Anspruch 9, wobei der mindestens eine Eisenchelator in einer Menge zugesetzt wird, so dass das Reagenz den mindestens einen Eisenchelator in einer Konzentration von 0,007 bis 2,5 mmol/L, besonders bevorzugt von 0,01 bis 0,5 mmol/L enthält.

11. Verfahren gemäß einem der Ansprüche 8 und 9, wobei das Siderophor ausgewählt ist aus der Gruppe Deferoxamin, Pyoverdin und Ferrichrom.

12. Verfahren gemäß einem der Ansprüche 8 und 9, wobei das 3,5-Diphenyl-1,2,4-Triazol ausgewählt ist aus der Gruppe Deferasirox, Ethyl[3,5-bis(2-hydroxyphenyl)-[1,2,4]triazol-1-yl]acetat und 3,5-Bis(2-hydroxyphenyl)-1-(2,2,2-trifluoroethyl)-1H-[1,2,4]triazol.

13. Verwendung eines Prothrombinzeit-Reagenzes gemäß einem der Ansprüche 1-7 in einem Verfahren zur Bestimmung eines Gerinnungsparameters in einer Plasmaprobe.

14. Verfahren zur Bestimmung eines Gerinnungsparameters in einer Plasmaprobe **dadurch gekennzeichnet, dass** die Plasmaprobe mit einem Prothrombinzeit-Reagenz gemäß einem der Ansprüche 1-7 zu einem Reaktionsgemisch vermischt wird und der Gerinnungsparameter in dem Reaktionsgemisch bestimmt wird.

15. Verfahren gemäß Anspruch 14 zur Bestimmung eines Gerinnungsparameters aus der Gruppe Prothombinzeit, abgeleitetes Fibrinogen und endogenes Thrombinpotenzial.

16. Verfahren gemäß Anspruch 14 zur Bestimmung der Prothombinzeit, wobei die Absorptionsänderung des Reaktionsgemisches photometrisch gemessen wird und die Zeit vom Zeitpunkt des Vermischens des Prothrombinzeit-Reagenzes mit der Plasmaprobe bis zur Erreichung eines Schwellwertes bestimmt wird.

17. Verwendung eines Eisenchelators aus der Gruppe Siderophor und 3,5-Diphenyl-1,2,4-Triazol zur Herstellung eines Prothrombinzeit-Reagenzes enthaltend Gewebefaktorprotein und Phospholipide.

18. Verwendung gemäß Anspruch 17, wobei das Siderophor ausgewählt ist aus der Gruppe Deferoxamin, Pyoverdin und Ferrichrom.

19. Verwendung gemäß Anspruch 17, wobei das 3,5-Diphenyl-1,2,4-Triazol ausgewählt ist aus der Gruppe Deferasirox, Ethyl[3,5-bis(2-hydroxyphenyl)-[1,2,4]triazol-1-yl]acetat und 3,5-Bis(2-hydroxyphenyl)-1-(2,2,2-trifluoroethyl)-1H-[1,2,4]triazol.

## Claims

1. Prothrombin time reagent comprising tissue factor protein and phospholipids, **characterized in that** the reagent further comprises at least one iron chelator, wherein the at least one iron chelator is selected from the group comprising siderophore and 3,5-diphenyl-1,2,4-triazole.

2. Prothrombin time reagent according to Claim 1, wherein the siderophore is selected from the group comprising deferoxamine, pyoverdine and ferrichrome.

3. Prothrombin time reagent according to Claim 1, wherein the 3,5-diphenyl-1,2,4-triazole is selected from the group comprising deferasirox, ethyl [3,5-bis(2-hydroxyphenyl)-[1,2,4]triazol-1-yl]acetate and 3,5-bis(2-hydroxyphenyl)-1-(2,2,2-trifluoroethyl)-1H-[1,2,4]triazole.

4. Prothrombin time reagent according to any of the preceding claims comprising the at least one iron chelator at a concentration of 0.007 to 2.5 mmol/L, particularly preferably 0.01 to 0.5 mmol/L.

5. Prothrombin time reagent according to any of the preceding claims, also comprising Ca²⁺ ions.

6. Prothrombin time reagent according to Claim 5 comprising 5 to 500 mmol/L calcium chloride.

7. Prothrombin time reagent according to any of the preceding claims, wherein the tissue factor protein is selected from the group of human or animal recombinant tissue factor protein and natural human or animal tissue factor protein from a tissue extract.

8. Method for preparing a liquid prothrombin time reagent comprising tissue factor protein and phospholipids and optionally Ca²⁺ ions, **characterized in that** a liquid comprising tissue factor protein, phospholipids and at least one iron chelator selected from the group comprising siderophore and 3,5-diphenyl-1,2,4-triazole is provided and the liquid is filled into a reagent vial without this liquid being lyophilized.

9. Method for stabilizing a prothrombin time reagent comprising tissue factor protein and phospholipids and optionally Ca²⁺ ions, **characterized in that** at least one iron chelator selected from the group comprising siderophore and 3,5-diphenyl-1,2,4-triazole is added to the reagent.

10. Method according to Claim 9, wherein the at least one iron chelator is added in an amount such that the reagent comprises the at least one iron chelator at a concentration of 0.007 to 2.5 mmol/L, particularly preferably 0.01 to 0.5 mmol/L.

11. Method according to either of Claims 8 and 9, wherein the siderophore is selected from the group comprising deferoxamine, pyoverdine and ferrichrome.

12. Method according to either of Claims 8 and 9, wherein the 3,5-diphenyl-1,2,4-triazole is selected from the group comprising deferasirox, ethyl [3,5-bis(2-hydroxyphenyl)-[1,2,4]triazol-1-yl]acetate and 3,5-bis(2-hydroxyphenyl)-1-(2,2,2-trifluoroethyl)-1H-[1,2,4]triazole.

13. Use of a prothrombin time reagent according to any of Claims 1-7 in a method for determining a coagulation parameter in a plasma sample.

14. Method for determining a coagulation parameter in a plasma sample, **characterized in that** the plasma sample is mixed with a prothrombin time reagent according to any of Claims 1-7 to give a reaction mixture and the coagulation parameter is determined in the reaction mixture.

15. Method according to Claim 14 for determining a coagulation parameter from the group of prothrombin time, derived fibrinogen and endogenous thrombin potential.

16. Method according to Claim 14 for determining the prothrombin time, wherein the change in absorption of the reaction mixture is measured photometrically and the time from the time point of mixing of the prothrombin time reagent with the plasma sample until reaching a threshold value is determined.

17. Use of an iron chelator from the group comprising siderophore and 3,5-diphenyl-1,2,4-triazole for preparing a prothrombin time reagent comprising tissue factor protein and phospholipids.

18. Use according to Claim 17, wherein the siderophore is selected from the group comprising deferoxamine, pyoverdine and ferrichrome.

19. Use according to Claim 17, wherein the 3,5-diphenyl-1,2,4-triazole is selected from the group comprising deferasirox, ethyl [3,5-bis(2-hydroxyphenyl)-[1,2,4]triazol-1-yl]acetate and 3,5-bis(2-hydroxyphenyl)-1-(2,2,2-trifluoroethyl)-1H-[1,2,4]triazole.

## Revendications

1. Réactif pour le temps de prothrombine, contenant une protéine de facteur de tissu et des phopholipides, **caractérisé en ce que** le réactif contient, en outre, au moins un chélateur du fer, le au moins un chélateur du fer étant choisi dans le groupe d'un sidérophore et d'un 3,5-diphényl-1,2,4-triazole.

2. Réactif pour le temps de prothrombine suivant la revendication 1, dans lequel le sidérophore est choisi dans le groupe de la déféroxamine, de la pyoverdine et du ferrichrome.

3. Réactif pour le temps de prothrombine suivant la revendication 1, dans lequel le 3,5-diphényl-1,2,4-triazole est choisi dans le groupe du déférasirox, du [3,5-bis(2-hydroxyphényl)-[1,2,4]-triazol-1-yl]acétate d'éthyle et du 3,5-bis(2-hydroxyphényl)-1-(2,2,2-trifluoroéthyl)-1H-[1,2,4]triazole.

4. Réactif pour le temps de prothrombine suivant l'une des revendications précédentes, **caractérisé en ce qu'**il contient au moins un chélateur du fer en une concentration de 0,007 à 2,5 mmol/L, d'une manière particulièrement préférée de 0, 01 à 0,5 mmol/L.

5. Réactif pour le temps de prothrombine suivant l'une des revendications précédentes, qui contient, en outre, des ions Ca²⁺.

6. Réactif pour le temps de prothrombine suivant la revendication 5, qui contient de 5 à 500 mmol/L de chlorure de calcium.

7. Réactif pour le temps de prothrombine suivant l'une des revendications précédentes, dans lequel la protéine de facteur de tissu est choisie dans le groupe d'une protéine de facteur de tissu recombinante, humaine ou animale et d'une protéine de facteur de tissu naturelle, humaine ou animale, à partir d'un extrait de tissu.

8. Procédé de préparation d'un réactif liquide pour le temps de prothrombine contenant une protéine de facteur de tissu et des phopholipides et, éventuellement, des ions Ca²⁺, **caractérisé en ce que** l'on se procure un liquide contenant une protéine de facteur de tissu, des phopholipides et au moins un chélateur du fer choisi dans le groupe d'un sidérophore et d'un 3,5-diphényl-1,2,4-triazole et on remplit un récipient de réaction du liquide sans lyophiliser ce liquide.

9. Procédé de stabilisation d'un réactif pour le temps de prothrombine, contenant une protéine de facteur de tissu et des phopholipides et, éventuellement des ions Ca²⁺, **caractérisé en ce que** l'on ajoute au réactif au moins un chélateur du fer choisi dans le groupe d'un sidérophore et d'un 3,5-diphényl-1,2,4 triazole.

10. Procédé suivant la revendication 9, dans lequel on joute le au moins un chélateur du fer en une quantité, de manière à ce que le réactif contienne le au moins un chélateur du fer en une concentration de 0,007 à 2,5 mmol/L, d'une manière particulièrement préférée de 0,01 à 0,5 mmol/L.

11. Procédé suivant l'une des revendications 8 et 9, dans lequel on choisit le sidérophore dans le groupe de la déféroxamine, de la pyoverdine et du ferrichrome.

12. Procédé suivant l'une des revendications 8 et 9, dans lequel on choisit le 3,5-diphényl-1,2,4-triazole dans le groupe du déféraxirox, du [3,5-bis(2-hydroxyphényl)-[1,2,4-triazol-1-yl]acétate d'éthyle et du 3,5-bis(2-hydroxyphényl)-1-(2,2,2-trifluoroéthyl)-1H[1,2,4]triazole.

13. Utilisation d'un réactif pour le temps de prothrombine suivant l'une des revendications 1 à 7, dans un procédé de détermination d'un paramètre de coagulation d'un échantillon de plasma.

14. Procédé de détermination d'un paramètre de coagulation d'un échantillon de plasma, **caractérisé en ce que** l'on mélange l'échantillon de plasma à un réactif pour le temps de prothrombine suivant l'une des revendications 1 à 7 en un mélange réactionnel et on détermine le paramètre de coagulation dans le mélange réactionnel.

15. Procédé suivant la revendication 14 de détermination d'un paramètre de coagulation choisi dans le groupe temps de prothrombine, fibrinogène dérivé et potentiel de thrombine endogène.

16. Procédé suivant la revendication 14 de détermination du temps de prothrombine, dans lequel on mesure photométriquement la variation d'absorption du mélange réactionnel et on détermine la durée allant de l'instant du mélange du réactif pour le temps de prothrombine à l'échantillon de plasma à l'atteinte d'une valeur de seuil.

17. Utilisation d'un chélateur du fer choisi dans le groupe d'un sidérophore et d'un 3,5-diphényl-1,2,4-triazol pour la préparation d'un réactif pour le temps de prothrombine contenant une protéine de facteur de tissu et des phopholipides.

18. Utilisation suivant la revendication 17, dans lequel le sidérophore est choisi dans le groupe de la déféroxamine, de la pyoverdine et du ferrichrome.

19. Utilisation suivant la revendication 17, dans lequel le 3,5-diphényle-1,2, 4-triazole est choisi dans le groupe du déférasirox, du [3,5-bis(2-hydroxyphényl)-[1,2,4]-triazol-1-yl]acétate d'éthyle et du 3,5-bis(2-hydroxyphényl)-1-(2,2,2-trifluoroéthyl)-1H-[1,2,4]triazole.
